# EUROPEAN PATENT SPECIFICATION

(11) **EP 2 054 894 B1**
(45) Date of publication and mention of the grant of the patent: **09.05.2018**
(21) Application number: 07804745.3
(22) Date of filing: 09.08.2007
(51) Int. Cl.: G21G 1/00, A61K 49/18, A61K 51/12, G01N 33/60, G01T 1/164, G21G 1/06, G01N 33/48, A61K 41/00, A61P 35/00

(54) **COMPOSITION APPARATUS AND METHOD FOR USE IN IMAGING**
ZUSAMMENSTELLUNGSVORRICHTUNG UND VERFAHREN ZUR VERWENDUNG BEI DER BILDGEBUNG
COMPOSITION APPAREILLAGE ET MÉTHODE SERVANT POUR L'IMAGERIE

(30) Priority: 11.08.2006 US 503418
(43) Date of publication of application: 06.05.2009
(60) Divisional application: 18163618.4
(73) Proprietor: Biocompatibles UK Limited, Farnham Surrey GU9 8QL (GB)
(72) Inventor: SIMPSON, Thomas, J., Ontario K2J 2E3 (CA); HAGERMAN, Jim, Québec J9J 1M4 (CA)
(74) Representative: Hyden, Martin Douglas
(86) International application number: PCT/IB2007/002307
(87) International publication number: WO 2008/017944

(56) References cited:
- WO-A1-00/44682
- WO-A1-00/64480
- WO-A1-91/10451
- WO-A1-91/10451
- WO-A1-97/01304
- WO-A1-97/01304
- WO-A2-96/40273
- WO-A2-2006/036269
- US-A1- 2004 242 953
- US-A1- 2006 204 443
- US-B1- 6 482 436
- US-B1- 6 482 436
- G RIUS ET AL: "An electron spin resonance study of the (OF) 2- molecular ion in neutron-irradiated 17 O-enriched MgO", JOURNAL OF PHYSICS C: SOLID STATE PHYSICS, vol. 7, no. 3, 7 February 1974 (1974-02-07), pages 581-585, XP55040502, ISSN: 0022-3719, DOI: 10.1088/0022-3719/7/3/013
- HAFELI U O: "MAGNETICALLY MODULATED THERAPEUTIC SYSTEMS", INTERNATIONAL JOURNAL OF PHARMACEUTICS, ELSEVIER BV, NL, vol. 277, no. 1/02, 11 June 2004 (2004-06-11), pages 19-24, XP008055248, ISSN: 0378-5173, DOI: 10.1016/J.IJPHARM.2003.03.002
- W.Y. LAU ET AL: "Selective internal radiation therapy for nonresectable hepatocellular carcinoma with intraarterial infusion of 90yttrium microspheres", INTERNATIONAL JOURNAL OF RADIATION ONCOLOGYBIOLOGYPHYSICS, vol. 40, no. 3, 1 February 1998 (1998-02-01), pages 583-592, XP55040496, ISSN: 0360-3016, DOI: 10.1016/S0360-3016(97)00818-3
- WEISSLEDER R: "Liver MR imaging with iron oxides: Toward consensus and clinical practice", RADIOLOGY, RADIOLOGICAL SOCIETY OF NORTH AMERICA, OAK BROOK,IL, US, vol. 193, no. 3, 1 December 1994 (1994-12-01), pages 593-595, XP008145207, ISSN: 0033-8419
- AHN J ET AL: "Hepatocellular carcinoma", DISEASE-A-MONTH, YEAR BOOK PUBL., CHICAGO, IL, US, vol. 50, no. 10, 1 October 2004 (2004-10-01), pages 556-573, XP004683249, ISSN: 0011-5029, DOI: 10.1016/J.DISAMONTH.2004.10.001

## Description

### Field

The subject matter of the present invention relates to imaging systems, such as magnetic resonance imaging (MRI) systems, and more particularly to detectable elements and radioactive materials for use in imaging systems and in therapy.

### Background

Radioactive microparticles infused into a subject, such as a human, and intended for delivery to a particular diseased organ can become trapped in organs other than the diseased organ. For example, radioactive microspheres infused into a subject and intended for delivery to a human liver can become trapped in the lungs of the subject. The entrapment of microspheres in the lungs is referred to as "lung shunt." At least two problems result from "lung shunt." First, the radiation dosage delivered to the diseased organ is less than intended, so the treatment may fail. Second, the radiation emitted by the radioactive microparticles trapped in the lungs can severely damage the lungs. Understanding the final distribution of radioactive microspheres in a subject's vasculature prior to treatment or during treatment can improve treatment results and prevent potentially catastrophic failure of the treatment.

### Brief Description of the Drawings

Figure 1(a) is an illustration of a composition comprising a microparticle including a radioactive isotope and an imageable element in accordance with some embodiments.
Figure 1(b) is an illustration of a composition comprising a microparticle including a radioactive isotope, and a dopant included in the microparticle in accordance with some embodiments.
Figure 2 is a flow diagram of a method of forming a microparticle including an enriched paramagnetic isotope and a radioactive isotope in accordance with some embodiments.
Figure 3 is a flow diagram of a method of forming a microparticle including an enriched paramagnetic isotope and Y-90 in accordance with some embodiments.
Figure 4 is a flow diagram of a method of selecting materials and forming a composition from the selected materials in accordance with some embodiments.
Figure 5(a) is a block diagram of an apparatus including an imaging system, a radioactive microparticle, shown in Figure 1(a) and Figure 1(b), and an enriched paramagnetic isotope in accordance with some embodiments.
Figure 5(b) is a block diagram of an apparatus including an imaging system, a microparticle that is not radioactive, and an enriched paramagnetic isotope in accordance with some embodiments.
Figure 6 is a flow diagram of a method of treating a disease and analyzing a disease state in accordance with some embodiments.
Figure 7 is a flow diagram of a method of analyzing a disease state in a subject after infusion of a detectable material into the subject in accordance with some embodiments.
Figure 8 is a flow diagram of a method of analyzing a disease state in a subject after infusion of an enriched paramagnetic isotope into the subject in accordance with some embodiments.

### Description

The present invention as claimed is limited to the subject matter of independent claims 1, 8, 11 and 12 and does not encompass further variants or embodiments disclosed herein.

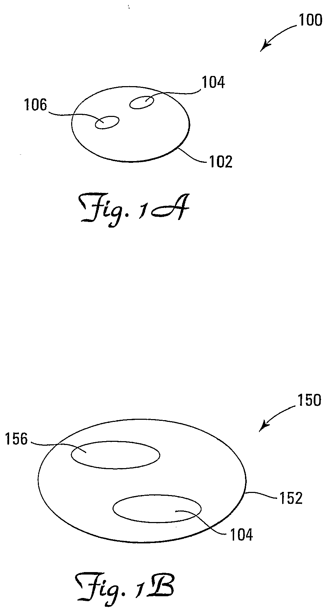

Figure 1(a) is an illustration of a composition 100 comprising a microparticle 102 including a radioactive isotope 104 and an imageable element 106 in accordance with some embodiments. The microparticle 102 including the radioactive isotope 104 and the imageable element 106 is suitable for use in connection with the treatment of disease and imaging. For example, the radioactive isotope 104 can be used in cancer treatments, and the imageable element 106 can be used to identify the location of the microparticle 102 in a subject, such as a cancer patient, after infusion. Patients include animals, such as mammals, including humans. Infusion includes infusion by a catheter or injection by a syringe. Methods of infusion are shown and described in United States Patent 4,745,907. The imageable element 106 is also suitable for use as a diagnostic tool in pre-treatment assessments or in conjunction with treatment of a disease.
For example, the imageable element 106 can be used to determine the distribution of the microparticles 102 within a subject after infusion. In applications in which the infused microparticles are intended for delivery to a human liver, the approximate percentage of microparticles delivered to the lung, which is sometimes referred to as a "lung shunt", can be determined through imaging. "Lung shunt" can vary among individuals. Thus, "lung shunt" information obtained through pre-treatment infusion and imaging at a substantially zero radioactive dosage level can be used to customize a treatment for a particular individual. The information can also be used to determine the radiation dose delivered to diseased tissue versus the radiation dose delivered to healthy tissue in a target organ which is also helpful in treatment planning. In one embodiment, the target organ is a human liver.

The microparticle 102 is not limited to having a particular shape or size. The shape of the microparticle 102 is selected for compatibility with the application in which the microparticle 102 is employed. For example, in pre-treatment evaluation applications, the shape of the microparticle 102 is selected to be substantially the same as the shape of the microparticle used in the treatment. In some embodiments, such as embodiments suitable for use in connection with cancer treatments, the microparticle 102 is substantially spherical.

The microparticle 102 has a size on the order of microns and can range from a fraction of a micron to thousands of microns. The size of the microparticle is selected for compatibility with the intended application. For example, an exemplary microparticle suitable for use in connection with a cancer treatment, such as a treatment for liver cancer, can have a diameter between about .1 micron and about 1000 microns. For pre-treatment applications, such as pre-treatment evaluations performed on animals, including humans, the microparticle 102 is substantially spherical and has a diameter substantially equal to the diameter of the microspheres used in the treatment. For diagnostic applications, the size of the microparticle is selected to achieve the desired results of the intended application.

A radioactive isotope of an element is a form of the element having an unstable nucleus that stabilizes itself by emitting radiation. The radioactive isotope 104 included in the microparticle 102 is not limited to a particular radioactive isotope. Exemplary radioactive isotopes suitable for use in connection with the microparticle 102 include the following therapeutic radioisotopes: As-211, P-32, Y-90, Cl-36, Re-186, Re-188, Au-198, Ho-166, I-131, Lu-177, P-33, Pr-147, Sc-47, Sr-89, S-35, I-125, Fe-55, and Pd-103.

The imageable element 106 is selected for compatibility with one or more imaging systems. For example, in some embodiments the imageable element 106 is selected for compatibility with a magnetic resonance imaging (MRI) system. Exemplary materials suitable for use in connection with imaging in a magnetic resonance imaging (MRI) system include paramagnetic materials and enriched paramagnetic materials or isotopes. In a paramagnetic material the atomic magnetic dipoles of the material have a tendency to align with an external magnetic field. A paramagnetic material exhibits magnetic properties such as experiencing a force when placed in a magnetic field. Exemplary paramagnetic materials suitable for use in connection with the formation of the composition 100 include H-1, He-3, Li-7, B-7, B-9, N-15, O-17, F-19, Mg-27, Al-27, Si-29, S-33, Cl-37, Ca-43, Ti-47, V-51, Cr-53, Mn-55, Fe-57, Ni-61, Cu-63, Zn-67, Ga-69, Ge-73, Kr-83, Sr-87, Y-89, Zr-91, Mo-95, Mo-97, Ru-99, Rh-103, Pd-105, Cd-111, Sn-115, Te-125, I-127, Ba-135, Ba-137, Xe-129, Xe-131, Nd-145, Gd-155, Dy-161, Er-167, Yb-171, W-183, Os-187, Pt-195, Hg-199, Tl-205, Pb-207, Pt-198, and H-2. The imageable element 106 can be incorporated into microparticles including those described in United States Patent 4,789,501 titled Glass Microspheres, United States Patent 5,011,677 titled Radioactive Glass Microspheres, United States Patent 5,011,797 titled Composition and Method for Radiation Synovectomy of Arthritic Joints, United States Patent 5,039,326 titled Composition and Method for Radiation Synovectomy of Arthritic Joints, United States Patent 5,302,369 Microspheres for Radiation Therapy, United States Patent 6,379,648 titled Biodegradable Glass Compositions and Methods for Radiation Therapy, and United States Patent 5,885,547 titled Particulate Material.

The microspheres or microparticles, in some embodiments, include essentially void-free glass microspheres, microshells, i.e., microspheres having a hollow core, or glass microspheres having a "foam-like" structure where the microsphere has a plurality of hollow cells. The microspheres or microparticles are not limited to a particular shape. In some embodiments, the microspheres or microparticles are substantially spherical, i.e., there are no sharp edges or points that would cause the microsphere to lodge in a location other than that desired. Thus, ellipsoidal and other similarly shaped particles that do not have sharp edges or points would be considered to be substantially spherical in shape. The microspheres may be processed to have a size that is appropriate for the therapeutic application.

The microspheres or microparticles may be prepared from a homogenous mixture of powders (i.e., the batch) that is melted to form the desired glass composition. The exact chemical compounds or raw materials used for the batch is not critical so long as they provide the necessary oxides in the correct proportion for the melt composition being prepared. After either dry or wet mixing of the powders to achieve a homogenous mixture, the mixture may be placed in a crucible for melting. The crucible containing the powdered batch is placed in a furnace which is heated to about 1500° to about 1600° C., depending upon the composition. In this temperature range, the batch melts to form a liquid which is stirred several times to improve its chemical homogeneity. The melt should remain at about 1500° to about 1600° C. till all solid material in the batch is dissolved. When melting and stirring is complete the crucible is removed from the furnace and the melt is quenched to a glass by pouring the melt onto a cold steel plate or into clean water. This procedure breaks the glass into fragments which aids and simplifies crushing the glass to a fine powder.

In some embodiments, the quenched and broken glass is crushed to about minus 100 mesh particles using a mortar and pestle. The minus 100 mesh material is ground until it passes a 400 mesh sieve. The particles are formed into glass microspheres by introducing the 400 mesh particles into a gas/oxygen flame where they are melted and a spherical liquid droplet is formed by surface tension. The droplets are rapidly cooled before they touch any solid object so that, their spherical shape is retained in the solid product.

In some embodiments, just prior to spheroidizing, the 400 mesh powder is rescreened through a 400 mesh sieve to destroy an large agglomerates that may have formed during storage. The powder is then placed in a vibratory feeder located above the gas/oxygen burner. The powder is slowly vibrated into a vertical glass tube which guides the falling powder particles directly into the hot flame of a gas/oxygen burner. The flame of the burner is directed into a metal container which catches the small glass beads as they are expelled from the flame. The container needs to be large enough so that the molten spheres can cool and become rigid before hitting any solid surface of the catcher container.

After spheroidization, the glass spheres are collected and rescreened. After the screening, the spheres are examined with an optical microscope and are then washed with a weak acid (HCl, for example), filtered and washed several times with reagent grade acetone. The washed spheres are then heated in a furnace in air to 500 degrees to 600 degrees C. for 2-6 hours to destroy any organic material. The glass spheres are examined in a scanning electron microscope to evaluate the size range and shape of the spheres.

An enriched paramagnetic material is a paramagnetic material in which the concentration of one or more isotopes of the paramagnetic material has been increased above the naturally occurring concentration. For example, the naturally occurring concentration of Gd-155 is about 14.8% in nature. Gd-155 is enriched when the concentration is increased to a concentration greater than about 14.8%. Methods of enrichment known to those skilled in the art of nuclear physics and chemistry and suitable for use in connection with the formation of the composition 100 include gaseous diffusion, chemical separation, electromagnetic separation, and laser separation. The preparation of the composition 100 is not limited to a particular enrichment or isotope separation method. The enrichment method is selected for compatibility with the materials selected to be enriched and to achieve the desired level of enrichment

The degree of enrichment of the imageable element 106 is not limited to a particular value. A higher level of enrichment results in increased signal strength and a higher resolution image. To increase signal strength for magnetic resonance imaging, Fe-57 is enriched to reduce radioactive impurities, Fe-54 and Fe-58 are substantially eliminated. Fe-54 can become a radioactive impurity with a single neutron capture. Fe-58 can become a radioactive impurity with a single neutron capture. In some embodiments, the imageable element 106 is enriched to a concentration of about 90%. In some embodiments, Fe-57 is enriched to a concentration of greater than about 92%. In some embodiments, Fe-57 is enriched to a concentration of about 92.88%. Enriching Fe-57 to a concentration beyond 90% increases the signal strength when compared to enrichment to a concentration of less than about 90%. In combination with increasing the concentration of the desired isotope in order to increase imaging resolution it is desirable to reduce the concentration of non-enriched isotopes to substantially zero. This lowers the probability of producing harmful isotopes when the microparticle 102 is activated. A harmful isotope is an isotope that is harmful to a subject or decreases the signal of the enriched element. Also disclosed herein are systems in which the imageable element 106 includes a radiopaque material. A radiopaque material is a material that is substantially opaque to at least some range of frequencies in the electromagnetic spectrum. Pb is one exemplary radiopaque material suitable for use as the imageable element 106 in a microparticle 102. Pb is substantially opaque to X-rays. Radiopaque materials, such as Pb, are imageable using computer-aided tomography (CT), fluoroscopy, and X-rays. The imageable element 106 can include Pb-206. Other radiopaque materials include enriched isotopes of Pb-207, Hg-198, Hg-199, Hg-200, Pt-195, Pt-198, Os-187, W-182, W-183, Yb-170, Yb-171, Yb-172, Er-166, Er-177, Dy-160, Dy-161, Dy-162, Dy-163, Gd-154, Gd-155, Gd-156, Cd-110, Cd-111, Sn-114, Sn-115, I-127, Ba-134, Ba-1.35, Ba-137, Ca-42, Ca-43, Mn-55, Fe-56, Fe-57, Ni-60, Ni-61, Zn-66, Zn-67, Zr-90, Zr-91, Mo-94, Mo-95, Mo-96, Mo-97, Ru-98, Ru-100, Rh-103, Pd-104, and Pd-105.

Pb-206 is an radiopaque isotope that could be used as the detectable element 106 in a composition 100. Pb-206 is approximately 24% abundant in nature. Pb-206 is three neutron captures away from Pb-209 which is the first significant radioactive impurity. The neutron absorption cross-sections for Pb-206, Pb-207, and Pb-208 are low, so the probability of neutron capture causing a harmful radioactive impurity is low. Although Pb-207 has a meta-stable state, Pb-207 also has a short half-life of about 0.8 seconds. The short half-life renders the meta-stable state substantially harmless. Pb-204 is 1.4% abundant and should be eliminated because a single neutron capture will yield Pb-205, which is a radioactive impurity with a long half-life. Enrichment of Pb-206 substantially eliminates the undesired isotopes, Pb-204, Pb-207, and Pb-208, and thus avoids the production of radioactive impurities during the activation process.

Figure 1(b) is an illustration of a composition 150 comprising a material 152 including a radioactive isotope 1.04, and a dopant 156 included in the material in accordance with some embodiments. The material 152 is not limited to a particular material or material in a particular form. Materials suitable for use in connection with the composition 150 can be in the form of solids, liquids, or gases. A dopant is a second material added to first material to change the first material's properties. For example, a paramagnetic material or isotope is added to a glass or ceramic substrate, such as a microsphere, to form a microsphere having paramagnetic properties. In some embodiments, the dopant 156 includes an enriched paramagnetic isotope. Exemplary enriched paramagnetic isotopes suitable for use in connection with the formation of the material 152 include H-1, He-3, Li-7, B-7, B-9, N-15, O-17, F-19, Mg-27, Al-27, Si-29, S-33, Cl-37, Ca-43, Ti-47, V-51, Cr-53, Mn-55, Fe-57, Ni-61, Cu-63, Zn-67, Ga-69, Ge-73, Kr-83, Sr-87, Y-89, Zr-91, Mo-95, Mo-97, Ru-99, Rh-103, Pd-105, Cd-111, Sn-115, Te-125, I-127, Ba-135, Ba-137, Xe-129, Xe-131, Nd-145, Gd-155, Dy-161, Er-167, Yb-171, W-183, Os-187, Pt-195, Hg-199, Tl-205, Pb-207, Pt-198, and H-2. The dopant 156 can be incorporated into microparticles including those described in United States Patent 4,789,501 titled Glass Microspheres, United States Patent 5,011,677 titled Radioactive Glass Microspheres, United States Patent 5,011,797 titled Composition and Method for Radiation Synovectomy of Arthritic Joints, United States Patent 5,039,326 titled Composition and Method for Radiation Synovectomy of Arthritic Joints, United States Patent 5,302,369 Microspheres for Radiation Therapy, United States Patent 6,379,648 titled Biodegradable Glass Compositions and Methods for Radiation Therapy, and United States Patent 5,885,547 titled Particulate Material. A method of making microparticles or microspheres suitable for use in connection with the formation of the detectable element 106 is described above.

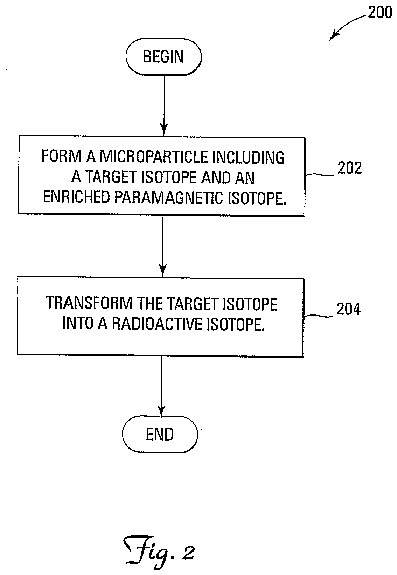

Figure 2 is a flow diagram of a method 200 of forming a microparticle including an enriched paramagnetic isotope and a radioactive isotope. The method 200 transforms a target isotope into a radioactive isotope. A target isotope is a stable isotope that becomes a radioactive isotope after absorbing a nuclear particle such as a neutron. The method 200 comprises forming a microparticle including a target isotope and an enriched paramagnetic isotope (block 202), and transforming the target isotope into a radioactive isotope (block 204). A microparticle produced by the method 200 includes a radioactive isotope and an enriched paramagnetic isotope. Thus, the microparticle can simultaneously treat diseased tissue with radiation and be imaged by an imaging system. In some embodiments, in forming a microparticle including a target isotope and an enriched paramagnetic isotope (block 202), an isotope that can be made radioactive through nuclear particle absorption is selected as the target isotope. Exemplary radioisotopes that can be produced using method 200 include At-211, P-32, Y-90, Cl-36, Re-186, Re-188, Au-198, Ho-166, I-131, Lu-177, P-33, Pr-147, Sc-47, Sr-89, S-35, I-125, Fe-55, and Pd-103. In some embodiments, in forming the microparticle including the target isotope and an enriched paramagnetic isotope, the enriched paramagnetic isotope is formed on a surface of the microparticle. The surface is the outer boundary of the microparticle. Methods of forming the enriched paramagnetic isotope on a surface of a microparticle include chemical vapor deposition, ion implantation, and chemical bonding. In some embodiments, in forming the microparticle including the target isotope and the enriched paramagnetic isotope, the enriched paramagnetic isotope is enriched to a concentration after enrichment of at least about 90%. Enrichment reduces the likelihood of formation of undesirable isotopes. In some embodiments, in transforming target isotope into the radioactive isotope, the target isotope is transformed into the radioactive isotope without forming a substantial number of undesired isotopes. In some embodiments, the method 200 further includes infusing the microparticle into living tissue to form a distribution of microparticles in the living tissue, and imaging the hydrogen near the microparticles.

Exemplary enriched paramagnetic isotopes suitable for use in connection with the method 200 include H-1, He-3, Li-7, B-7, B-9, N-15, O-17, F-19, Mg-27, Al-27, Si-29, S-33, Cl-37, Ca-43, Ti-47, V-51, Cr-53, Mn-55, Fe-57, Ni-61, Cu-63, Zn-67, Ga-69, Ge-73, Kr-83, Sr-87, Y-89, Zr-91, Mo-95, Mo-97, Ru-99, Rh-103, Pd-105, Cd-111, Sn-115, Te-125, I-127, Ba-135, Ba-137, Xe-129, Xe-131, Nd-145, Gd-155, Dy-161, Er-167, Yb-171, W-183, Os-187, Pt-195, Hg-199, Tl-205, Pb-207, Pt-198, and H-2. Exemplary particles suitable for use in irradiating the target isotope include neutrons, protons, and heavier particles, such as deuterium+, tritium+, and helium++.

The method 200 is not limited to forming particles of a particular shape. In some embodiments, forming the microparticle including the target isotope includes forming a substantially spherical microparticle. Substantially spherical microparticles are suitable for use in connection with the treatment of cancers, such as liver cancer.

The method 200 reduces radioactive impurities while maintaining or increasing the strength of the magnetic resonance imaging signal through enrichment of the paramagnetic isotope. Failure to reduce radioactive impurities can result in tissue damage through radiation burning or radiation induced cell damage resulting in cancer. The method 200 is not limited to a particular degree of enrichment. In some embodiments, doping the microparticle with the enriched paramagnetic isotope includes doping the microparticle with an enriched paramagnetic isotope having a concentration after enrichment of at least about 90%. In some embodiments, transforming the target isotope into the radioactive isotope includes transforming the target isotope into the radioactive isotope without forming a substantial number of impurity isotopes. The number of impurity isotopes are substantial for a particular treatment when the number prevents safe, convenient, and effective use of the treatment.

In some embodiments, the method 200 further includes infusing the microparticle into living tissue to form a distribution of microparticles in the living tissue, and imaging the hydrogen near the microparticles affected by the paramagnetic elements in the microparticles. Magnetic resonance imaging (MRI) scanning instruments can be setup to measure the response from the paramagnetic element in the microparticle, or to measure the response of adjacent hydrogen that has been affected by the paramagnetic element.

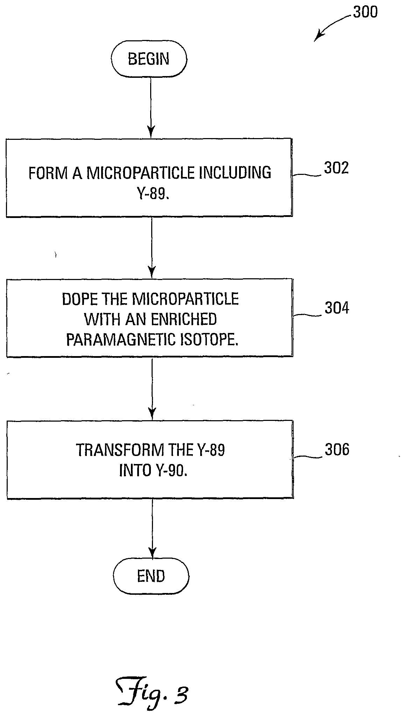

Figure 3 is a flow diagram of a method 300 of forming a microparticle including an enriched paramagnetic isotope and Y-90 in accordance with the claimed invention. The method 300 includes forming a microparticle including Y-89 (block 302), doping the microparticle with an enriched paramagnetic isotope (block 304), and transforming the Y-89 into Y-90 (block 306). In some embodiments, the method 300 further includes infusing the microparticle into living tissue, such as human tissue, to form a distribution of microparticles in the living tissue, and imaging the distribution of microparticles to provide information for analyzing the distribution of the microparticles. One purpose of the analysis is to understand the distribution and concentration of microparticles in a target organ. The distribution and concentration information can be used to determine the dosage level delivered to the lesions in a target organ, as well as to the healthy tissue in the target organ. This particle distribution information can be used to optimize the dose size as well as the number of particles used to deliver the dose. The particle distribution can subsequently be used to determine radiation dose characteristics in the tissue. The particle distribution can also be used to determine relative doses for healthy versus diseased tissue. Another purpose of the analysis is to understand the distribution and concentration of microparticles in tissue other than the target organ. Information related to the distribution and concentration of microparticles in tissue other than the target organ is useful in determining whether the intended dosage was delivered to the lesions in the target organ and whether unacceptable amounts of material were delivered to other organs. In yet other embodiments, imaging the distribution of microparticles to provide information for analyzing the distribution of the microparticles includes using magnetic resonance imaging to image the distribution of microparticles. In some embodiments, the Y-89 is transformed into Y-90 through an (n,gamma) reaction in a nuclear reactor.

In the method 300, in some embodiments for neutron activated Y-90 microspheres the doping element is enriched with paramagnetic isotopes, such as Fe-57 or Gd-155. A result of the enrichment process is that radioactive impurities, isotopes other than the paramagnetic isotopes that would be activated if present at activation time, are substantially eliminated. This method is effective when the neutron absorption cross-section for the paramagnetic isotope in the doping material is close to or less than the cross-section of Y-89, and when more than one neutron capture is required for the creation of a radioactive impurity.

For example, Gd-155 is a paramagnetic isotope that is four neutron captures away from forming the harmful radioactive impurity Gd-159. Although the neutron absorption cross-sections for the Gd isotopes are higher than the neutron absorption cross-sections for Y-89, the probability of four neutron captures is low in the period of time it takes to convert the Y-89 into Y-90 in a nuclear reactor or other neutron source. In another example, Fe-57 is two neutron captures away from forming the harmful radioactive impurity Fe-59. The neutron absorption cross-section of Fe-57 and Fe-58 are low, so the probability of a double neutron capture is low in the time it takes to produce Y-90 from Y-89 in a nuclear reactor or other neutron source.

Y-89 is transformed to Zr-89 after a (p,n) reaction and provides an improved signal in positron emission tomography (PET). In some embodiments, the method 300 further includes transforming Y-89 to Zr-89 for use in connection with positron emission tomography (PET). Other useful PET isotopes that can be formed by absorbing a neutron particle include Cu-64 and Zr-89. Exemplary radioisotopes suitable for use in connection with positron emission tomograph (PET) include F-18, I-124, and Sr-85.

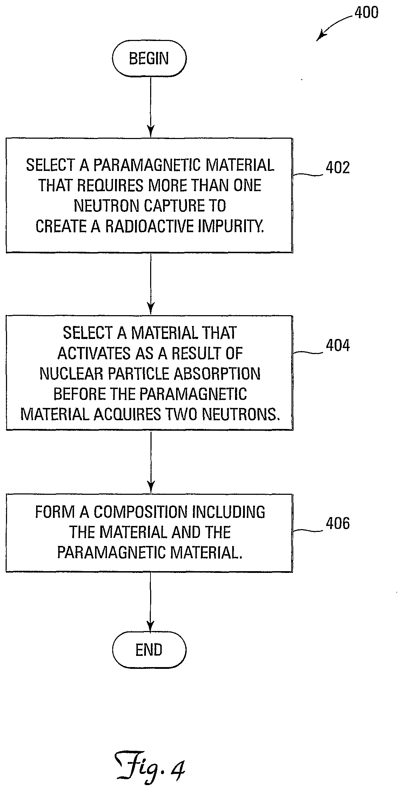

Figure 4 is a flow diagram of a method 400 of selecting materials and forming a composition from the selected materials. The method 400 includes selecting a paramagnetic material that requires more than one neutron capture to create a radioactive impurity (block 402), selecting a material that activates as a result of nuclear particle absorption before the paramagnetic material acquires two neutrons (block 404), and forming a composition including the material and the paramagnetic material (block 406). Neutron activation is the process by which neutron radiation induces radioactivity in materials. Neutron activation occurs when nuclei capture free neutrons. The nuclei become heavier nuclei in excited states, so the material that includes the heavier nuclei becomes radioactive.

In some embodiments, the method 400 further includes introducing the composition into a subject, such as a human. In some embodiments, the method including introducing the composition into a subject further includes forming an image of the composition and the subject by magnetic resonance imaging (MRI). In other embodiments, the method including introducing the composition into the subject further includes forming and analyzing an image of the composition to determine whether a disease is present in the subject. The method 400 can also include treating a disease with the composition, and forming an image of the composition using an imaging system. In some embodiments, forming the composition including the material and the paramagnetic material includes activating the composition through nuclear particle absorption.

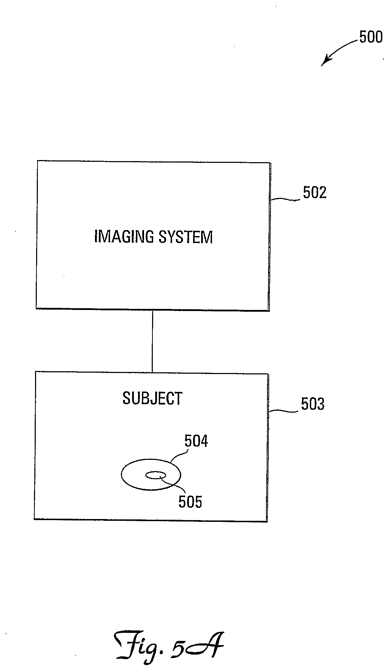

Figure 5(a) is a block diagram of an apparatus 500 including an imaging system 502 to image a subject 503, and a radioactive microparticle 504 suitable for infusion into the subject 503 for imaging by the imaging system and including an enriched paramagnetic isotope 505 that is enriched to reduce generation of radioactive impurities while maintaining or improving imaging sensitivity. The imaging system 502 is not limited to a particular type of imaging system. Exemplary imaging systems and methods suitable for use in connection with the apparatus 500 include magnetic resonance imaging (MRI), computer-aided tomography (CT), single photon emission computed tomography (SPECT), positron emission tomography (PET), and fluoroscopy.

Magnetic resonance imaging (MRI) includes the use of a nuclear magnetic resonance spectrometer to produce electronic images of atoms and molecular structures in solids, including human cells, tissues, and organs. Computer-aided tomography (CT) includes the generation of a three-dimensional image of the internals of a subject or object from a plurality of two-dimensional X-ray images taken around a single axis of rotation. Single photon emission computed tomography (SPECT) includes a tomographic imaging technique using gamma rays and produces a set of image slices through a subject, showing the distribution of a radiopharmaceutical or radioactive particle. Positron emission tomography (PET) includes producing a three dimensional image or map of functional processes in the body. X-ray tomography produces a series of projection images used to calculate a three-dimensional reconstruction of an object. Fluoroscopy includes producing real-time images of the internal structures of a subject through the use of a fluoroscope. A fluoroscope produces fluorescent images of a patient on a fluorescent screen by imaging the subject using X-rays.

The apparatus 500 is not limited to use in connection with a particular type of subject. Organic and inorganic materials can be imaged by the apparatus 500. Exemplary subjects also include living tissue, including live animals, and dead tissue, including preserved tissue and non-preserved tissue. The enriched paramagnetic isotope is not limited to a particular paramagnetic isotope. In some embodiments, the enriched paramagnetic isotope includes a material capable of neutron activation having a first neutron absorption cross-section, and a paramagnetic isotope having a second neutron absorption cross-section within a factor of about 1000 of the first neutron absorption cross-section and which requires more than one neutron capture to create a radioactive impurity.

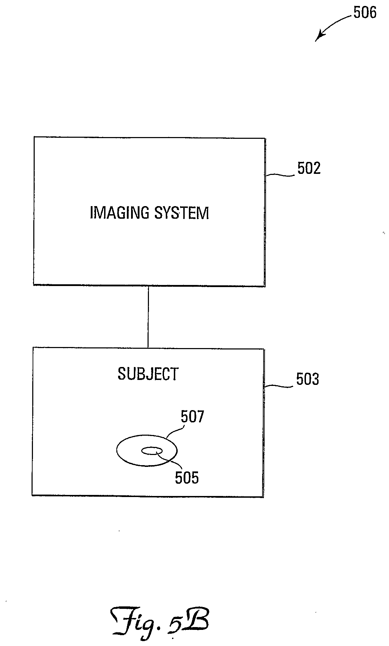

Figure 5(b) is a block diagram of an apparatus 506 including the imaging system 502 to image the subject 503, and a microparticle 507 suitable for infusion into the subject 503 for imaging by the imaging system 502 and including the enriched paramagnetic isotope 505 that is enriched to reduce generation of radioactive impurities while maintaining or improving imaging sensitivity. The imaging system 502 is not limited to a particular type of imaging system. Exemplary imaging systems and methods suitable for use in connection with the apparatus 506 include magnetic resonance imaging (MRI), computer-aided tomography (CT), single photon emission computed tomography (SPECT), positron emission tomography (PET), and fluoroscopy. The microparticle 507 is not radioactive.

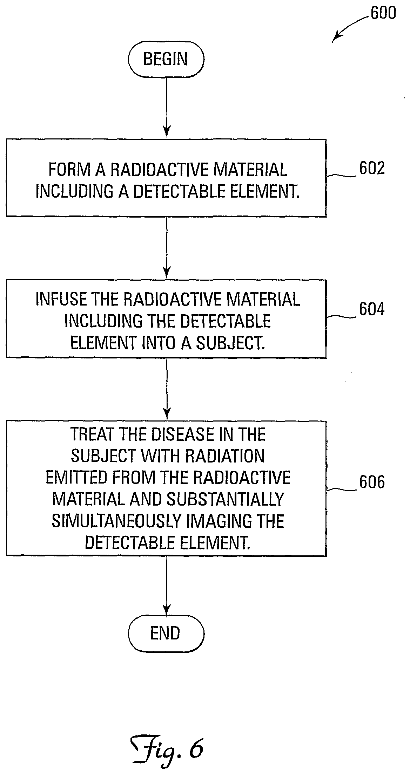

Figure 6 is a flow diagram of a method 600 of treating a disease and analyzing a disease state in accordance with some embodiments. The method 600 includes forming a radioactive material including a detectable element (block 602), infusing the radioactive material including the detectable element into a subject (block 604), and treating a disease in the subject with radiation emitted from the radioactive material and substantially simultaneously imaging the detectable element (block 606). In some embodiments, forming the radioactive material including the detectable element includes forming the radioactive material through activation by nuclear particle absorption. In some embodiments, forming the radioactive material through activation by nuclear particle absorption includes forming the radioactive material by absorption of neutrons, protons, particles heavier than protons, deuterium+, tritium+, or helium++. In some embodiments, imaging the detectable element includes imaging the detectable element using computer-aided tomography (CT). In some embodiments, imaging the detectable element includes imaging the detectable element using fluoroscopy. In some embodiments, imaging the detectable element includes imaging the detectable element using positron emission tomography (PET). In some embodiments, infusing the radioactive material including the paramagnetic isotope into the subject having the disease includes infusing the radioactive material including the radioactive isotope into a living animal.

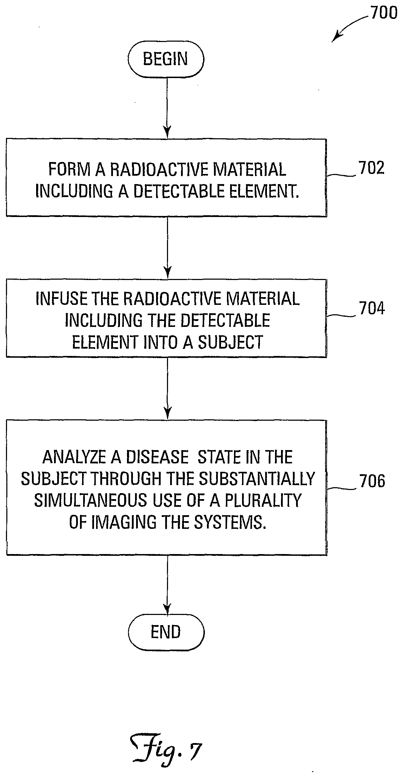

Figure 7 is a flow diagram of a method 700 of analyzing a disease state in a subject after infusion of a detectable material into the subject in accordance with some embodiments. The method 700 includes forming a radioactive material including a detectable element (block 702), infusing the radioactive material including the detectable element into a subject (block 704), and analyzing a disease state in the subject through the substantially simultaneous use of a plurality of imaging systems (block 706). In some embodiments, forming the radioactive material including the detectable element includes forming the radioactive material through activation by nuclear particle absorption after forming the radioactive material including the detectable element.

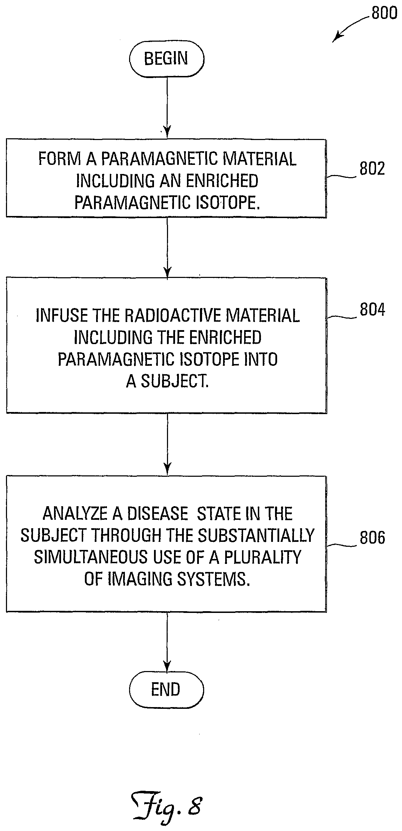

Figure 8 is a flow diagram of a method 800 for analyzing a disease state in a subject after infusion of an enriched paramagnetic isotope into the subject in accordance with some embodiments. The method 800 includes forming a radioactive material including an enriched paramagnetic isotope (block 802), infusing the radioactive material including the enriched paramagnetic isotope into the subject (block 804), and analyzing a disease state in the subject through the substantially simultaneous use of a plurality of imaging systems (block 806). In some embodiments, forming the radioactive material including the paramagnetic isotope includes forming the radioactive material through activation by nuclear particle absorption. In some embodiments, infusing the radioactive material including the paramagnetic isotope into the subject includes delivering the radioactive material including the paramagnetic isotope in the form of one or more microspheres to the subject where the subject includes a mammal. In some embodiments, analyzing the disease state in the subject through the substantially simultaneous imaging using a plurality of imaging systems includes analyzing the disease state using a magnetic resonance imaging (MRI) and single photon emission computed tomography (SPECT). Exemplary SPECT imageable isotopes include Cu-67, Ga-67, Ho-166, In-111, I-123, I-131, Lu-177, Gd-153, Kr-85, and Xe-133.

## Claims

1. A composition comprising:
a microparticle including:
a radioactive isotope; and
an imageable element comprising an enriched paramagnetic material in which the concentration of one or more isotopes of the paramagnetic material have been increased above the naturally occurring concentration;
**characterised in that**:
the radioactive isotope is Y-90, producible by transformation of Y-89 target isotope in the microparticle by neutron activation; and
more than one neutron capture is required for the creation of a radioactive impurity from the paramagnetic isotope.

2. The composition of claim 1, wherein the paramagnetic isotope has a neutron absorption cross-section less than that of Y-89.

3. The composition of claim 1, wherein the enriched paramagnetic material is enriched to a concentration after enrichment of at least 90%.

4. The composition of any one of the preceding claims, wherein the microparticle comprises a microsphere.

5. The composition of any one of the preceding claims, wherein the paramagnetic isotope comprises Fe-57, Gd-155, or O-17.

6. The composition of any one of the preceding claims:
wherein the imageable element is a dopant.

7. The composition of any preceding claim, wherein the enriched paramagnetic material is formed on the surface of the microparticle.

8. A method for preparing a composition comprising a glass microparticle, said method comprising:
forming a microparticle including a target isotope and an enriched paramagnetic material in which the concentration of one or more isotopes of the paramagnetic material have been increased above the naturally occurring concentration; and
transforming the target isotope into a radioactive isotope;
**characterised in that**
the target isotope is Y-89 that is transformed into the radioactive isotope Y-90 by neutron activation; and
more than one neutron capture is required for the creation of a radioactive impurity from the paramagnetic isotope.

9. The method of claim 8, wherein the neutron absorption cross-section for the paramagnetic isotope is less than that of Y-89.

10. The method of claim 8 or 9, wherein forming the microparticle including the target isotope and the enriched paramagnetic material comprises:
forming the enriched paramagnetic material on a surface of the microparticle.

11. A composition as defined in any one of claims 1 to 7 for use in therapy.

12. An apparatus for imaging a composition as defined in any one of claims 1 to 7 comprising:
an imaging system to image a subject; and
a radioactive microparticle as defined in any one of claims 1 to 7, suitable for infusion into the subject for imaging by the imaging system and including an enriched paramagnetic material that is enriched to reduce generation of radioactive impurities while maintaining or improving imaging sensitivity.

## Patentansprüche

1. Zusammensetzung, umfassend:
ein Mikropartikel, beinhaltend:
ein radioaktives Isotop; und
ein abbildbares Element, welches ein angereichertes paramagnetisches Material umfasst, wobei die Konzentration von einem oder mehreren Isotopen des paramagnetischen Materials über die natürlich auftretende Konzentration hinaus erhöht worden ist;
**dadurch gekennzeichnet, dass**:
das radioaktive Isotop Y-90 ist, das durch Transformation von Y-89-Zielisotop in dem Mikropartikel durch Neutronenaktivierung produziert werden kann; und
mehr als ein Neutronenfang für die Erzeugung einer radioaktiven Verunreinigung aus dem paramagnetischen Isotop benötigt wird.

2. Zusammensetzung nach Anspruch 1, wobei das paramagnetische Isotop einen Neutronenabsorptionsquerschnitt aufweist, der kleiner als der von Y-89 ist.

3. Zusammensetzung nach Anspruch 1, wobei das angereicherte paramagnetische Material auf eine Konzentration nach der Anreicherung von mindestens 90 % angereicht wird.

4. Zusammensetzung nach einem der vorhergehenden Ansprüche, wobei das Mikropartikel ein Mikrokügelchen umfasst.

5. Zusammensetzung nach einem der vorhergehenden Ansprüche, wobei das paramagnetische Isotop Fe-57, Gd-155 oder 0-17 umfasst.

6. Zusammensetzung nach einem der vorhergehenden Ansprüche:
wobei das abbildbare Element ein Dotiermittel ist.

7. Zusammensetzung nach einem der vorhergehenden Ansprüche, wobei das angereicherte paramagnetische Material auf der Oberfläche des Mikropartikels ausgebildet wird.

8. Verfahren zum Herstellen einer Zusammensetzung, die ein Glasmikropartikel umfasst, wobei das Verfahren Folgendes umfasst:
Ausbilden eines Mikropartikels, das ein Zielisotop und ein angereichertes paramagnetisches Material beinhaltet, wobei die Konzentration von einem oder mehreren Isotopen des paramagnetischen Materials über die natürlich auftretende Konzentration hinaus erhöht worden ist; und
Transformieren des Zielisotops in ein radioaktives Isotop;
**dadurch gekennzeichnet, dass**
das Zielisotop Y-89 ist, das durch Neutronenaktivierung in das radioaktive Isotop Y-90 transformiert wird; und
mehr als ein Neutronenfang für die Erzeugung einer radioaktiven Verunreinigung aus dem paramagnetischen Isotop benötigt wird.

9. Verfahren nach Anspruch 8, wobei der Neutronenabsorptionsquerschnitt für das paramagnetische Isotop kleiner als der von Y-89 ist.

10. Verfahren nach Anspruch 8 oder 9, wobei Ausbilden des Mikropartikels, welches das Zielisotop und das angereicherte paramagnetische Material beinhaltet, Folgendes umfasst:
Ausbilden des angereicherten paramagnetischen Materials auf einer Oberfläche des Mikropartikels.

11. Zusammensetzung nach einem der Ansprüche 1 bis 7 zur Verwendung in der Therapie.

12. Vorrichtung zum Darstellen einer Zusammen nach einem der Ansprüche 1 bis 7, umfassend:
ein Bildgebungssystem zum Darstellen eines Subjekts; und
ein radioaktives Mikropartikel nach einem der Ansprüche 1 bis 7, das für die Infusion in das Subjekt zum Darstellen durch das Bildgebungssystem geeignet ist und ein angereichertes paramagnetisches Material beinhaltet, das angereichter ist, um die Generierung radioaktiver Verunreinigungen zu reduzieren, während die Bildgebungsempfindlichkeit beibehalten oder verbessert wird.

## Revendications

1. Composition comprenant :
une microparticule comprenant :
un isotope radioactif ; et
un élément imageable comprenant un matériau paramagnétique enrichi dans lequel la concentration d'un ou de plusieurs isotopes du matériau paramagnétique a été augmentée au-dessus de la concentration naturelle ;
**caractérisée en ce que** :
l'isotope radioactif est Y-90, pouvant être produit par transformation de l'isotope cible Y-89 dans la microparticule par activation neutronique ; et
plus d'une capture de neutrons est requise pour la création d'une impureté radioactive à partir de l'isotope paramagnétique.

2. Composition selon la revendication 1, dans laquelle l'isotope paramagnétique a une section transversale d'absorption de neutrons inférieure à celle de Y-89.

3. Composition selon la revendication 1, dans laquelle le matériau paramagnétique enrichi est enrichi à une certaine concentration après enrichissement d'au moins 90 %.

4. Composition selon l'une quelconque des revendications précédentes, dans laquelle la microparticule comprend une microsphère.

5. Composition selon l'une quelconque des revendications précédentes, dans laquelle l'isotope paramagnétique comprend Fe-57, Gd-155 ou 0-17.

6. Composition selon l'une quelconque des revendications précédentes :
dans laquelle l'élément imageable est un dopant.

7. Composition selon une quelconque revendication précédente, dans laquelle le matériau paramagnétique enrichi est formé sur la surface de la microparticule.

8. Procédé de préparation d'une composition comprenant une microparticule de verre, ledit procédé comprenant :
la formation d'une microparticule comprenant un isotope cible et un matériau paramagnétique enrichi dans lequel la concentration d'un ou de plusieurs isotopes du matériau paramagnétique a été augmentée au-dessus de la concentration naturelle ; et
la transformation de l'isotope cible en un isotope radioactif ;
**caractérisé en ce que**
l'isotope cible est Y-89 qui est transformé en l'isotope radioactif Y-90 par activation neutronique ; et
plus d'une capture de neutrons est requise pour la création d'une impureté radioactive à partir de l'isotope paramagnétique.

9. Procédé selon la revendication 8, dans lequel la section transversale d'absorption de neutrons pour l'isotope paramagnétique est inférieure à celle de Y-89.

10. Procédé selon la revendication 8 ou 9, dans lequel la formation de la microparticule comprenant l'isotope cible et le matériau paramagnétique enrichi comprend :
la formation du matériau paramagnétique enrichi sur une surface de la microparticule.

11. Composition selon l'une quelconque des revendications 1 à 7 destinée à être utilisée dans une thérapie.

12. Appareillage pour l'imagerie d'une composition telle que définie selon l'une quelconque des revendications 1 à 7 comprenant :
un système d'imagerie pour former une image d'un sujet ; et
une microparticule radioactive telle que définie selon l'une quelconque des revendications 1 à 7, conçue pour une perfusion chez le sujet pour l'imagerie par le système d'imagerie et comprenant un matériau paramagnétique enrichi qui est enrichi pour réduire la génération d'impuretés radioactives tout en maintenant ou en améliorant la sensibilité d'imagerie.
